# EUROPEAN PATENT APPLICATION

(11) **EP 4 609 874 A1**
(43) Date of publication of application: **03.09.2025**
(21) Application number: 23881878.5
(22) Date of filing: 25.10.2023
(51) Int. Cl.: A61K 38/12, A61K 9/08, A61K 9/19, A61K 9/10, A61K 47/22, A61K 47/26, A61P 31/10

(54) **PHARMACEUTICAL COMPOSITION COMPRISING ECHINOCANDIN ANALOG AND PREPARATION METHOD FOR PHARMACEUTICAL COMPOSITION**

(30) Priority: 25.10.2022 CN 202211312487; 07.07.2023 CN 202310830843
(71) Applicant: Jiangsu Hengrui Pharmaceuticals Co., Ltd., Lianyungang, Jiangsu 222047 (CN)
(72) Inventor: FAN, Yingfang, Lianyungang, Jiangsu 222047 (CN); YE, Linmao, Lianyungang, Jiangsu 222047 (CN); CHEN, Hao, Lianyungang, Jiangsu 222047 (CN); AN, Dong, Lianyungang, Jiangsu 222047 (CN); MENG, Qingqing, Lianyungang, Jiangsu 222047 (CN); JIANG, Kai, Lianyungang, Jiangsu 222047 (CN)
(74) Representative: dompatent von Kreisler Selting Werner - Partnerschaft von Patent- und Rechtsanwälten mbB
(86) International application number: PCT/CN2023/126450
(87) International publication number: WO 2024/088301

(57) **Abstract**

The present disclosure provides a pharmaceutical composition comprising an echinocandin analog and a preparation method for the pharmaceutical composition. Specifically, the present disclosure provides a pharmaceutical composition, comprising a compound represented by formula (I) or a pharmaceutically acceptable salt thereof, a solubilizer, and a buffer. The composition has excellent stability.

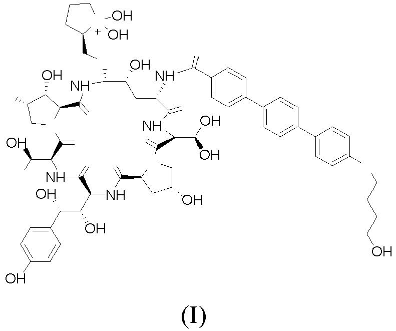

## Description

The present application claims priority to Chinese patent application 2022113124878 filed on October 25, 2022 and Chinese patent application 2023108308433 filed on July 7, 2023. The contents of the above Chinese patent applications are incorporated herein by reference in their entirety.

### TECHNICAL FIELD

The present disclosure belongs to the field of pharmaceutical preparations, and specifically relates to a pharmaceutical composition comprising an echinocandin analog and a preparation method therefor.

### BACKGROUND

Currently available drugs for the treatment of fungal infections include amphotericin B, a macrolide polyene that interacts with fungal membrane sterols; flucytosine, a fluoropyrimidine that interacts with fungal protein and DNA biosynthesis; and a variety of azole antifungals (e.g., ketoconazole, itraconazole, and fluconazole) that inhibit fungal membrane-sterol biosynthesis (Alexander et al., Drugs, 54:657, 1997). The application of amphotericin B is limited due to infusion-related reactions and nephrotoxicity, in spite of the fact that it has a broad spectrum of activity and is considered the "gold standard" for antifungal therapy (Warnock, J. Antimicrob. Chemother., 41:95, 1998). The use of flucytosine is also limited due to the development of drug-resistant microorganisms and its narrow spectrum of activity. The widespread use of azole antifungals is leading to the emergence of clinically resistant strains of *Candida spp.*

Echinocandins are a novel class of antifungals that are typically comprised of a cyclic hexapeptide and a lipophilic tail, the latter of which is attached to the hexapeptide core via an amide linkage. These drugs interfere with the synthesis of β-1,3-glucose in fungal cell walls by non-competitive inhibition of β-1,3-glucose synthase, leading to changes in the permeability of the fungal cell walls, and thus cell lysis and death. Due to the absence of cell walls in human cells and the presence of cell walls in fungal cells, echinocandin antifungals can directly act upon the components of the fungal cell walls, thereby having low toxicity to humans. Therefore, these drugs have been one of the safest antifungals to date.

Anidulafungin is a class of echinocandin drugs developed by Vicuron Pharmaceuticals (USA) and marketed under the brand name Eraxis, which was approved by the FDA in 2006. Anidulafungin has a broad spectrum of antimicrobial activity. CN100335122C discloses a pharmaceutical composition comprising anidulafungin and a micelle-forming surfactant.

WO2021110125 discloses a novel-structure echinocandin antifungal, such as a compound represented by formula I, which demonstrates excellent antimicrobial activity.

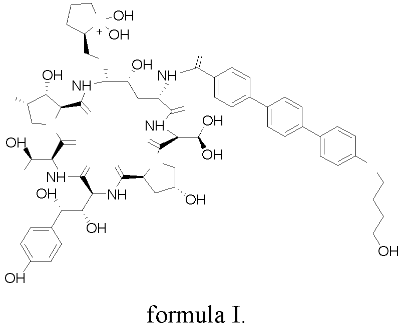

### SUMMARY

The present disclosure aims to provide a pharmaceutical composition comprising an active ingredient of a compound represented by formula I or a pharmaceutically acceptable salt thereof, wherein the composition has good formulation stability.

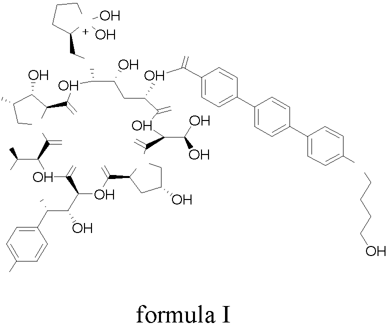

The present disclosure provides a pharmaceutical composition comprising a compound represented by formula I as an active ingredient or a pharmaceutically acceptable salt thereof, a solubilizer, and a buffer, wherein the buffer is selected from one or more of glycine, alanine, valine, leucine, isoleucine, phenylalanine, serine, threonine, tyrosine, cysteine, methionine, proline, tryptophan, lysine, arginine, histidine, aspartic acid, glutamic acid, asparagine, glutamine, phosphate, citrate, maleate, borate, carbonate, acetate, lactate, tartrate, malate, succinate, ascorbate, oxalate, and sulfate.

The buffer may be in free form or salt form, or a combination of both.

The present disclosure provides a pharmaceutical composition comprising a compound represented by formula I as an active ingredient or a pharmaceutically acceptable salt thereof, a solubilizer, and a buffer, wherein the buffer comprises at least one amino acid.

In certain embodiments, the solubilizer is selected from one or more of polysorbate, polyoxyethylene castor oil derivatives, polyoxyethylene stearate, sorbitan trioleate, bile salts, and lecithin. In certain embodiments, the solubilizer is polysorbate, such as polysorbate 20, polysorbate 40, polysorbate 60, or polysorbate 80. The solubilizer facilitates preventing precipitation of the active ingredient during storage or *in vivo* after administration, thereby reducing potential side effects (e.g., irritation).

In certain embodiments, the buffer is histidine, including histidine and a combination of histidine and histidine hydrochloride.

In certain embodiments, the pharmaceutical composition may further comprise a stabilizer, wherein the stabilizer is selected from one or more of mannitol, sucrose, trehalose, maltose, dextrose, and lactose. In certain embodiments, the stabilizer is mannitol.

In certain embodiments, the pharmaceutical composition may further comprise a pH adjuster, wherein the pH adjuster may be sodium hydroxide, hydrochloric acid, *etc.*

In certain embodiments, the compound represented by formula I or the pharmaceutically acceptable salt thereof is an acetate of the compound represented by formula I.

In certain embodiments, the weight ratio of the compound represented by formula I as an active ingredient or the pharmaceutically acceptable salt thereof to the buffer is 1:0.01 to 1:5. In certain embodiments, the weight ratio of the compound represented by formula I as an active ingredient or the pharmaceutically acceptable salt thereof to the buffer is 1:0.05 to 1:5, non-limiting examples of which include 1:0.01, 1:0.02, 1:0.05, 1:0.1, 1:0.15, 1:0.2, 1:0.25, 1:0.3, 1:0.35, 1:0.4, 1:0.45, 1:0.5, 1:0.6, 1:0.7, 1:0.8, 1:0.9, 1:1, 1:1.5, 1:2, 1:2.5, 1:3, 1:3.5, 1:4, 1:4.5, 1:5, or any value between any two of these ratios. In certain embodiments, the weight ratio of the compound represented by formula I as an active ingredient or the pharmaceutically acceptable salt thereof to the buffer is 1:0.02 to 1:3, such as 1:0.05 to 1:1, which is suitable for stabilizing the pH of the composition.

In certain embodiments, the weight ratio of the compound represented by formula I as an active ingredient or the pharmaceutically acceptable salt thereof to the solubilizer is 1:0.1 to 10. In certain embodiments, the weight ratio of the compound represented by formula I as an active ingredient or the pharmaceutically acceptable salt thereof to the solubilizer is 1:0.5 to 5, non-limiting examples of which include 1:1, 1:1.2, 1:1.4, 1:1.5, 1:1.6, 1:1.8, 1:2, 1:2.2, 1:2.4, 1:2.5, 1:2.6, 1:2.8, 1:3, 1:3.2, 1:3.4, 1:3.5, 1:3.6, 1:3.8, 1:4, 1:4.2, 1:4.4, 1:4.5, 1:4.6, 1:4.8, 1:5, or any value between any two of these ratios.

In certain embodiments, the weight ratio of the compound represented by formula I as an active ingredient or the pharmaceutically acceptable salt thereof to the stabilizer is 1:0.1 to 10, preferably 1:0.5 to 5, non-limiting examples of which include 1:0.5, 1:0.6, 1:0.8, 1:0.9, 1:1, 1:1.2, 1:1.4, 1:1.6, 1:1.8, 1:2, 1:2.2, 1:2.4, 1:2.6, 1:2.8, 1:3, 1:3.2, 1:3.4, 1:3.6, 1:3.8, 1:4, 1:4.2, 1:4.4, 1:4.6, 1:4.8, 1:5, or any value between any two of these ratios.

In certain embodiments, the pharmaceutical composition has a pH of 4 to 8. In certain embodiments, the pharmaceutical composition has a pH of 4.5 to 7. In certain embodiments, the pharmaceutical composition has a pH of 5.5 to 6.5, non-limiting examples of which include 4.5, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, or any value between any two of these numbers.

In certain embodiments, the content of the active ingredient or the pharmaceutically acceptable salt thereof in the pharmaceutical composition is 5 to 300 mg/mL, such as 10 to 250 mg/mL, non-limiting examples of which include 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250 mg/mL, or any value between any two of these numbers.

The present disclosure further provides a pharmaceutical composition comprising:
5 to 300 mg/mL of a compound represented by formula I or a pharmaceutically acceptable salt thereof;
a stabilizer, which is mannitol, wherein the weight ratio of the compound represented by formula I or the pharmaceutically acceptable salt thereof to the stabilizer is 1:0.5 to 5;
a buffer, which is histidine, wherein the weight ratio of the compound represented by formula I or the pharmaceutically acceptable salt thereof to the buffer is 1:0.05 to 5;
a solubilizer, which is polysorbate, wherein the weight ratio of the compound represented by formula I or the pharmaceutically acceptable salt thereof to the solubilizer is 1:0.5 to 5;
and optionally a pH adjuster,
wherein the composition has a pH of 5.5 to 6.5.

The pharmaceutical composition of the present disclosure may further comprise other pharmaceutical excipients, such as cosolvents, tonicity adjusters, adsorbents, or complexing agents.

The pharmaceutical composition provided by the present disclosure may be in solid or liquid form, wherein the pharmaceutical composition in solid form is typically obtained by lyophilizing a composition in liquid form, or may be redissolved or reconstituted to obtain a composition in liquid form. The pharmaceutical composition in liquid form may be in the form of a solution or a suspension, preferably a solution. In certain embodiments, the liquid composition is obtained by redissolving or reconstituting a lyophilized composition or is a liquid composition prior to lyophilization. In certain embodiments, the solid composition is redissolved or reconstituted to obtain the aforementioned pharmaceutical composition.

The present disclosure provides a use of the aforementioned pharmaceutical composition in the manufacture of a medicament for treating and/or preventing fungal infections.

The present disclosure provides a use of the aforementioned pharmaceutical composition in the manufacture of a medicament for preventing, stabilizing, or inhibiting fungal growth or killing fungi.

The pharmaceutical composition provided by the present disclosure is used as a medicament. The medicament may be used to prevent, stabilize, or inhibit fungal growth or kill fungi.

The present disclosure provides a method for preventing, stabilizing, or inhibiting fungal growth or killing fungi, comprising administering a therapeutically effective amount of the pharmaceutical composition to a patient in need thereof.

The present disclosure provides a method for preparing the aforementioned pharmaceutical composition, comprising the step of mixing a compound represented by formula I or a pharmaceutically acceptable salt thereof with a solubilizer and a buffer.

In certain embodiments, the method further comprises the steps of pH adjustment and lyophilization.

The terms "about" and "approximately" as used herein mean that a numerical value is within an acceptable error range for the particular value as determined by one of ordinary skill in the art, and the numerical value depends in part on how the value is measured or determined (*i.e.,* the limitations of the measurement system). For example, "about" may mean within 1 or more than 1 standard deviation per the practice in the art. Alternatively, "about" or "substantially comprising" may mean a range of up to 20%. Furthermore, particularly for biological systems or processes, the term may mean up to an order of magnitude or up to 5-fold of a numerical value. Unless otherwise stated, when a particular value is provided in the present application and claims, the meaning of "about" or "substantially comprising" should be assumed to be within an acceptable error range for the particular value.

The numerical values in the present disclosure are instrument measurements or calculated values based on instrument measurements, and have a certain degree of error. Generally, ±10% falls within a reasonable error range. It is certainly necessary to consider the context in which the numerical value is used. For example, for the content of total impurities, the error range of the value after measurement shall not exceed ±10%, and may be ±9%, +8%, ±7%, ±6%, ±5%, ±4%, ±3%, ±2%, or ±1%, preferably ±5%.

The term "weight-to-volume ratio" in the present disclosure refers to the weight (g) of the ingredient per 100 mL of the liquid system, *i.e.,* g/100 mL.

### DETAILED DESCRIPTION OF THE PREFERRED EMBODIMENT

The following examples are provided to further illustrate the present disclosure in detail. These examples are provided merely for illustrative purposes and are not intended to limit the scope of the present disclosure.

In the examples, the content of acetate of the compound represented by formula I is calculated based on the free form of the compound represented by formula I.

### Example 1: Buffer screening experiment

Experimental method: Tween 80 was weighed and mixed uniformly with water for injection until dissolved. The active ingredient was then added and stirred to dissolve. A buffer was weighed and dissolved in water for injection with stirring, and mixed uniformly with the active ingredient solution. Mannitol was then added, and the pH was adjusted to 5.5 to 6.5. The mixture was sterilized by filtration, filled into vials, and lyophilized to prepare compositions 2 and 3, respectively.

**Table 1: Components of each composition**

| | Composition 1 | Composition 2 | Composition 3 |
|---|---|---|---|
| Acetate of compound represented by formula I | 200 mg | 200 mg | 200 mg |
| Tween 80 | 500 mg | 400 mg | 500 mg |
| Mannitol | 200 mg | 200 mg | 200 mg |
| Acetic acid | 6 mg | | |
| Histidine | / | 21 mg | 21 mg |
| Histidine hydrochloride | / | 42 mg | 42 mg |
| Hydrochloric acid/sodium hydroxide | q.s. | q.s. | q.s. |
| pH | 5.5 to 6.5 | 5.5 to 6.5 | 5.5 to 6.5 |
| Water for injection | 4 mL | 4 mL | 4 mL |

Test conclusion: Composition 1 exhibited poor stability with a significant increase in pH after lyophilization. Compositions 2 and 3, which employed a pH buffer system of histidine/histidine hydrochloride, maintained the pH of the redissolved solution within the range of 5.5 to 6.5.

**Table 2: Buffer screening results**

| No. | pH before lyophilization | pH of redissolved solution after lyophilization |
|---|---|---|
| Composition 1 | 6.3 | 8.3 |
| Composition 2 | 5.9 | 6.4 |
| Composition 3 | 6.0 | 6.4 |

### Example 2: Formulation stability

Tween 80 was weighed and mixed uniformly with water for injection until dissolved. The active ingredient was then added and stirred to dissolve. A buffer was weighed and dissolved in water for injection with stirring, and mixed uniformly with the active ingredient solution. Mannitol was then added, and the pH was adjusted to 5.5 to 6.5. The mixture was sterilized by filtration, filled into vials, and lyophilized to prepare compositions A and B.

**Table 3: Components of each composition**

| | Composition A | Composition B |
|---|---|---|
| Acetate of compound represented by formula I | 200 mg | 200 mg |
| Tween 80 | 400 mg | 500 mg |
| Mannitol | 200 mg | 200 mg |
| Histidine | 21 mg | 21 mg |
| Histidine hydrochloride | 42 mg | 42 mg |
| Hydrochloric acid/sodium hydroxide | q.s. | q.s. |
| pH | 5.5 to 6.5 | 5.5 to 6.5 |
| Water for injection | 4 mL | 4 mL |

**Experimental design and protocol:** Compositions A and B were subjected to stress testing to evaluate the effects of temperature (40°C ± 2°C/RH 75% ± 5%), freeze-thaw cycles (2 days at -20°C followed by 2 days at 25°C as 1 cycle), and light exposure on the samples. The specific protocol is shown in Table 4, and results are shown in Table 5.

**Table 4: Stress testing protocol**

| **Placement conditions** | | **Testing timepoints** | **Testing parameters** |
|---|---|---|---|
| High temperature | 40°C ± 2°C/RH 75% ± 5% | 0 days, 5 days, 12 days, 30 days | Appearance; clarity and color of solution; pH; moisture; visible particles; insoluble particulates; related substances; content |
| Freeze-thaw cycle | 2 days at -20°C followed by 2 days at 25°C as 1 cycle | 3 cycles | |
| Light exposure | Illuminance = 1.2*10⁶ Lux·hr Near-UV energy ≥ 200 w·hr/m² | 0 days, 5 days, 12 days, 30 days | |

**Table 5.1: Stress testing results - high temperature results**

| **Testing parameters** | **Composition A** | | | | **Composition B** | | | |
|---|---|---|---|---|---|---|---|---|
| | **Initial point** | **High temperature for 5 days** | **High temperature for 12 days** | **High temperature for 30 days** | **Initial point** | **High temperature for 5 days** | **High temperature for 12 days** | **High temperature for 30 days** |
| Appearance | Off-white lyophilized cake | Off-white lyophilized cake | Off-white lyophilized cake | Off-white lyophilized cake | Off-white lyophilized cake | Off-white lyophilized cake | Off-white lyophilized cake | Off-white lyophilized cake |
| Clarity and color of solution | Clear, colorless | / | I | Clear, colorless | Clear, colorless | / | / | Clear, colorless |
| pH | 6.4 | / | / | 6.4 | 6.4 | / | / | 6.4 |
| Total impurities | 0.35% | 0.37% | 0.43% | 0.73% | 0.34% | 0.37% | 0.43% | 0.71% |
| Isomer | 0.26% | 0.27% | 0.28% | 0.29% | 0.25% | 0.27% | 0.28% | 0.30% |
| Content determination (on a free base basis) | 105.4% | 105.0% | 103.5% | 105.3% | 103.0% | 103.5% | 103.9% | 103.1% |

| | | | | | | | | |
|---|---|---|---|---|---|---|---|---|
| **[0052]** Note: "/" indicates that the parameter is not tested at the timepoint. | | | | | | | | |

**Table 5.2: Stress testing results - freeze-thaw cycle results**

| **Testing parameters** | **Composition A** | | | | **Composition B** | | | |
|---|---|---|---|---|---|---|---|---|
| | **Initial point** | **Cycle 1** | **Cycle 2** | **Cycle 3** | **Initial point** | **Cycle 1** | **Cycle 2** | **Cycle 3** |
| Appearance | Off-white lyophilized cake | Off-white lyophilized cake | Off-white lyophilized cake | Off-white lyophilized cake | Off-white lyophilized cake | Off-white lyophilized cake | Off-white lyophilized cake | Off-white lyophilized cake |
| Clarity and color of solution | Clear, colorless | / | / | Clear, colorless | Clear, colorless | / | / | Clear, colorless |
| pH | 6.4 | / | / | 6.4 | 6.4 | / | / | 6.4 |
| Total impurities | 0.35% | 0.36% | 0.32% | 0.32% | 0.34% | 0.37% | 0.34% | 0.32% |
| Isomer | 0.26% | / | 0.27% | 0.27% | 0.25% | 0.27% | 0.27% | 0.27% |
| Content determination | 105.4% | 105.3% | 103.9% | 105.1% | 103.0% | 103.4% | 103.6% | 103.4% |

| | | | | | | | | |
|---|---|---|---|---|---|---|---|---|
| **[0054]** Note: "/" indicates that the parameter is not tested at the timepoint. | | | | | | | | |

**Table 5.3: Stress testing results - light exposure results (composition A)**

| **Testing parameters** | **Initial point** | **Light exposure of unboxed vials** | | | **Light exposure of vials in box packaging** | | |
|---|---|---|---|---|---|---|---|
| | | **5 days** | **12 days** | **30 days** | **5 days** | **12 days** | **30 days** |
| Appearance | Off-white lyophilized cake | Off-white lyophilized cake | Off-white lyophilized cake | Off-white lyophilized cake | Off-white lyophilized cake | Off-white lyophilized cake | Off-white lyophilized cake |
| Clarity and color of solution | Clear, colorless | / | / | Clear, colorless | / | / | Clear, colorless |
| pH | 6.4 | / | / | 6.4 | / | / | 6.4 |
| Total impurities | 0.35% | 0.35% | 0.34% | 0.43% | 0.34% | 0.34% | 0.41% |
| Isomer | 0.26% | 0.27% | 0.27% | 0.27% | 0.27% | 0.27% | 0.27% |
| Content determination | 105.4% | 105.0% | 103.8% | 105.0% | 105.4% | 104.5% | 105.2% |

| | | | | | | | |
|---|---|---|---|---|---|---|---|
| **[0056]** Note: "/" indicates that the parameter is not tested at the timepoint. | | | | | | | |

**Table 5.4: Stress testing results - light exposure results (composition B)**

| **Testing parameters** | **Initial point** | **Light exposure of unboxed vials** | | | **Light exposure of vials in box packaging** | | |
|---|---|---|---|---|---|---|---|
| | | **5 days** | **12 days** | **30 days** | **5 days** | **12 days** | **30 days** |
| Appearance | Off-white lyophilized cake | Off-white lyophilized cake | Off-white lyophilized cake | Off-white lyophilized cake | Off-white lyophilized cake | Off-white lyophilized cake | Off-white lyophilized cake |
| Clarity and color of solution | Clear, colorless | / | / | Clear, colorless | / | / | Clear, colorless |
| pH | 6.4 | / | / | 6.4 | / | / | 6.4 |
| Total impurities | 0.34% | 0.34% | 0.32% | 0.41% | 0.33% | 0.34% | 0.41% |
| Isomer | 0.25% | 0.27% | 0.27% | 0.27% | 0.27% | 0.27% | 0.27% |
| Content determination | 103.0% | 103.6% | 103.1% | 104.2% | 103.4% | 103.9% | 103.7% |

| | | | | | | | |
|---|---|---|---|---|---|---|---|
| Note: "/" indicates that the parameter is not tested at the timepoint. | | | | | | | |

**Experimental conclusion:** Both compositions demonstrated good stability under all stress conditions.

### Example 3

Using the method described in Example 1, comparative compositions were prepared with different buffers according to the components in Table 6, and the pH of each composition was measured before lyophilization and after redissolution.

**Table 6: Components of each composition**

| | Formula 1 | Formula 2 | Formula 3 | Formula 4 | Formula 5 |
|---|---|---|---|---|---|
| Acetate of compound represented by formula I | 200 mg | 200 mg | 200 mg | 200 mg | 200 mg |
| Tween 80 | 400 mg | 400 mg | 400 mg | 400 mg | 400 mg |
| Mannitol | 200 mg | 200 mg | 200 mg | 200 mg | 200 mg |
| Glycine | / | / | 11.3 mg | / | / |
| Arginine hydrochloride | / | / | / | 63 mg | |
| Lactic acid | 0.5 mg | / | / | / | / |
| Tartaric acid | / | 1.83 mg | / | / | / |
| Citric acid | / | / | / | / | 68 mg |
| Hydrochloric acid/sodium hydroxide | q.s. to pH 5.5 to 6.5 | | | | |
| Water for injection | 4 mL | 4 mL | 4 mL | Until dissolved | 4 mL |
| pH before lyophilization | 5.96 | 5.65 | 5.53 | 5.54 | 5.68 |
| pH of redissolved solution after lyophilization | 8.05 | 8.05 | 8.66 | 8.54 | 8.10 |

**Experimental conclusion:** Other types of pH buffers showed inferior buffering capacity compared to histidine on the pH of the compositions.

### Example 4

Using the method described in Example 1, compositions were prepared with different levels of excipients according to the components in Table 7, and the pH of each composition was measured before lyophilization and after redissolution.

**Table 7: Components of each composition**

| | Composition 4 | Composition 5 | Composition 6 |
|---|---|---|---|
| Acetate of compound represented by formula I | 200 mg | 200 mg | 200 mg |
| Tween 80 | 400 mg | 400 mg | 450 mg |
| Mannitol | 200 mg | 450 mg | 500 mg |
| Histidine | 37 mg | 21 mg | 21 mg |
| Histidine hydrochloride | / | 42 mg | 42 mg |
| Hydrochloric acid/sodium hydroxide | q.s. | q.s. | q.s. |
| pH | 5.5 to 6.5 | 5.5 to 6.5 | 5.5 to 6.5 |
| Water for injection | 4 mL | 4 mL | 4 mL |
| pH before lyophilization | 5.47 | 5.9 | 5.9 |
| pH of redissolved solution after lyophilization | 6.45 | 6.5 | 6.4 |

**Experimental conclusion:** The excipients showed good buffering capacity on the pH of each composition.

## Claims

1. A pharmaceutical composition comprising a compound represented by formula I or a pharmaceutically acceptable salt thereof, a solubilizer, and a buffer, wherein the buffer is selected from one or more of glycine, alanine, valine, leucine, isoleucine, phenylalanine, serine, threonine, tyrosine, cysteine, methionine, proline, tryptophan, lysine, arginine, histidine, aspartic acid, glutamic acid, asparagine, glutamine, phosphate, citrate, maleate, borate, carbonate, acetate, lactate, tartrate, malate, succinate, ascorbate, oxalate, and sulfate, more preferably histidine;

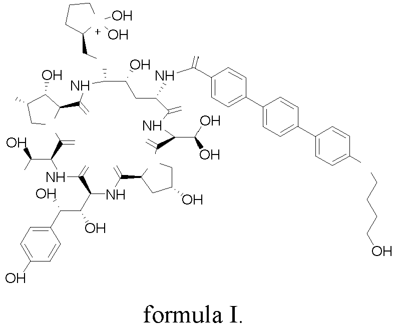

2. The pharmaceutical composition according to claim 1, wherein the solubilizer is selected from one or more of polysorbate, polyoxyethylene castor oil derivatives, polyoxyethylene stearate, sorbitan trioleate, bile salts, and lecithin, preferably polysorbate.

3. The pharmaceutical composition according to claim 1 or 2, further comprising a stabilizer, wherein the stabilizer is selected from one or more of mannitol, sucrose, trehalose, maltose, dextrose, and lactose, more preferably mannitol.

4. The pharmaceutical composition according to any one of claims 1 to 3, further comprising a pH adjuster.

5. The pharmaceutical composition according to any one of claims 1 to 4, wherein the compound represented by formula I or the pharmaceutically acceptable salt thereof is an acetate of the compound represented by formula I.

6. The pharmaceutical composition according to any one of claims 1 to 5, wherein the weight ratio of the compound represented by formula I or the pharmaceutically acceptable salt thereof to the buffer is 1:0.01 to 5, preferably 1:0.05 to 5.

7. The pharmaceutical composition according to any one of claims 1 to 6, wherein the weight ratio of the compound represented by formula I or the pharmaceutically acceptable salt thereof to the solubilizer is 1:0.1 to 10, preferably 1:0.5 to 5.

8. The pharmaceutical composition according to any one of claims 1 to 7, wherein the weight ratio of the compound represented by formula I or the pharmaceutically acceptable salt thereof to the stabilizer is 1:0.1 to 10, preferably 1:0.5 to 5.

9. The pharmaceutical composition according to any one of claims 1 to 8, wherein the composition has a pH of 4 to 8, preferably 4.5 to 7, more preferably 5.5 to 6.5.

10. The pharmaceutical composition according to any one of claims 1 to 9, wherein the content of the compound represented by formula I or the pharmaceutically acceptable salt thereof is 5 to 300 mg/mL, preferably 10 to 250 mg/mL.

11. A pharmaceutical composition comprising:
5 to 300 mg/mL of a compound represented by formula I or a pharmaceutically acceptable salt thereof;
a stabilizer, which is mannitol, wherein the weight ratio of the compound represented by formula I or the pharmaceutically acceptable salt thereof to the stabilizer is 1:0.5 to 5;
a buffer, which is histidine, wherein the weight ratio of the compound represented by formula I or the pharmaceutically acceptable salt thereof to the buffer is 1:0.05 to 5;
a solubilizer, which is polysorbate, wherein the weight ratio of the compound represented by formula I or the pharmaceutically acceptable salt thereof to the solubilizer is 1:0.5 to 5;
and optionally a pH adjuster,
wherein the composition has a pH of 5.5 to 6.5.

12. The pharmaceutical composition according to any one of claims 1 to 11, wherein the pharmaceutical composition is a liquid composition.

13. A solid composition, wherein the solid composition is redissolved or reconstituted to obtain the pharmaceutical composition as defined in claims 1 to 12.

14. A lyophilized composition, wherein the lyophilized composition is obtained by lyophilizing the pharmaceutical composition as defined in any one of claims 1 to 12.

15. A redissolved solution, wherein the redissolved solution is prepared by redissolving the solid composition as defined in claim 13 or the lyophilized composition as defined in claim 14.

16. Use of the pharmaceutical composition as defined in any one of claims 1 to 12, the solid composition as defined in claim 13, the lyophilized composition as defined in claim 14, or the redissolved solution as defined in claim 15 in the manufacture of a medicament for treating and/or preventing fungal infections.

17. Use of the pharmaceutical composition as defined in any one of claims 1 to 12, the solid composition as defined in claim 13, the lyophilized composition as defined in claim 14, or the redissolved solution as defined in claim 15 in the manufacture of a medicament for preventing, stabilizing, or inhibiting fungal growth or killing fungi.

18. A method for preparing the pharmaceutical composition as defined in any one of claims 1 to 13, comprising the step of mixing a compound represented by formula I or a pharmaceutically acceptable salt thereof with a solubilizer and a buffer.

19. The method according to claim 18, further comprising the step of lyophilization.
